# EUROPÄISCHE PATENTANMELDUNG

(11) **EP 0 966 976 A1**
(43) Veröffentlichungstag der Anmeldung: **29.12.1999**
(21) Anmeldenummer: 99111573.4
(22) Anmeldetag: 15.06.1999
(51) Int. Cl.: A61L 2/00

(54) **Verfahren zum Abtrennen von HIV aus einer Flüssigkeit**

(30) Priorität: 22.06.1998 DE 19827750
(71) Anmelder: Centeon Pharma GmbH, 35002 Marburg (DE)
(72) Erfinder: Gröner, Albrecht, Dr., 64342 Seeheim (DE); Römisch, Jürgen, Dr., 35041 Marburg (DE)

(57) **Zusammenfassung**

Es wird ein Verfahren zum Abtrennen von HIV aus einer Flüssigkeit beschrieben, bei dem die HIV an einen auf einem Trägermaterial fixierten C1-Esterase-Inhibitor gebunden werden. Das Verfahren kann sowohl zur Bereitstellung HIV-freier Blutkonserven als auch therapeutisch zur Reduktion der Viruslast im Blut durch eine Blutwäsche unter den Bedingungen eines extrakorporalen Blutkreislaufes erfolgen. Der C1-Esterase-Inhibitor kann an einem in der Affinitätschromatographie üblichen Trägermaterial oder an den Fasern eines Filters gebunden werden.

## Beschreibung

Gegenstand der Erfindung ist ein Verfahren zum Abtrennen von Humanem Immundefizienz Virus bzw. Viren (HIV) aus einer Flüssigkeit, insbesondere aus Blut, Blutplasma oder Blutserum. Das Verfahren kann sowohl zur Bereitstellung HIV-freier Blutkonserven als auch therapeutisch zur Reduktion der Viruslast im Blut durch eine Blutwäsche unter den Bedingungen eines extrakorporalen Blutkreislaufes erfolgen. Außerdem ist die Erfindung auf einen Filter gerichtet, der zur Abtrennung von HIV aus einer Flüssigkeit geeignet ist.

Es ist bekannt, daß die Entfernung von HIV aus den verschiedensten biologischen Flüssigkeiten, vor allem aber aus Blut, Blutplasma oder Blutserum eine wichtige Voraussetzung für ihren risikolosen Einsatz für die verschiedensten medizinischen Zwecke ist. Es sind deshalb auch schon zahlreiche Verfahren vorgeschlagen worden, mit denen eine Entfernung von HIV aus biologischen Flüssigkeiten erreicht werden soll. So ist in der internationalen Patentanmeldung WO 97/07674 ein Verfahren vorgeschlagen worden, mit dem HIV aus biologischen Flüssigkeiten entfernt oder inaktiviert werden können, in dem man sie mit bestimmten Ethylenimin-Oligomeren behandelt. Wichtig ist dabei, daß andere Bestandteile des Blutes, insbesondere die zellulären Bestandteile, vor allem die Erythrozyten, durch eine derartige Behandlung nicht beschädigt werden und die Entfernung der HIV in einfacher Weise und kurzer Zeit durchgeführt werden kann, damit genügend große Mengen gereinigten Blutes in einem wirtschaftlich vertretbaren Verfahren gewonnen werden können.

Es wurde nun gefunden, daß diese Anforderungen in hervorragender Weise durch ein Verfahren erfüllt werden können, wenn zur Entfernung der HIV aus biologischen Flüssigkeiten der C1-Inhibitor eingesetzt wird.

Der C1-Inhibitor, auch als C1-Esterase-Inhibitor bezeichnet, ist ein im Blut vorhandenes Protein und ist der Hauptinhibitor des klassischen Weges des Komplementsystems und des Kontaktsystems. Der C1-Inhibitor kann die aktivierte Form des Faktors XII und des Kallikrein hemmen (Schapira M. et al., 1985, Complement 2 111; Davis A.E., 1988, Ann Rev Immunol 6: 595; Sim R.B. et al., 1979, FEBS Lett 97: 111; De Agostini A. et al., 1984, J Clin Invest 73: 1542; Pixley R.A. et al., 1985, J Biol Chem 260: 1723; Schapira M. et al., 1982, J Clin Invest 69: 462; Van der Graaf F. et al., 1983, J Clin Invest 71: 149; Harpel P.C. et al., 1975, J Clin Invest 55: 593). Der C1-Inhibitor reguliert somit die Aktivitäten von zwei Plasmakaskaden, nämlich das Komplementsystem und das Kontaktsystem, durch die biologisch aktive Peptide erzeugt werden. Der C1-Inhibitor ist deshalb auch ein wichtiger Regulator des Entzündungssystems. Außerdem hemmt der C1-Inhibitor den aktivierten Faktor XI (Meijers J.C.M. et al., 1988, Biochemistry 27: 959; Wuillemin W.A. et al., 1995, Blood 85: 1517). Hieraus ergibt sich, daß der C1-Inhibitor als ein Coagulations-Hemmstoff betrachtet werden kann. Auch der Gewebeplasminogen-Aktivator und Plasmin werden in gewissem Ausmaß von dem C1-Inhibitor gehemmt, obwohl das nicht seine Hauptfunktion ist (Harpel P.C. et al., 1975, J Clin Invest 55: 149; Booth N.A. et al., 1987, Blood 69: 1600).

Der C1-Inhibitor wird in erheblichem Ausmaß durch Reinigung aus Plasma gewonnen und für klinische Anwendungen benutzt, insbesondere bei der Behandlung des hereditären Angioödems, einer Erkrankung, die durch einen genetisch bedingten Mangel des C1-Inhibitors hervorgerufen wird. Außerdem ist bereits beschrieben worden, daß durch Verabreichung des C1-Inhibitors bei systemischen Entzündungen [Internationale Patentanmeldung WO 92/22320 (Genentech Inc.)], bei schweren Verbrennungen, Pankreatitis, Knochenmarkstransplantationen, der Zytokin-Therapie und beim Einsatz in extrakorporalen Blutkreisläufen [DE-A-4 227 762 (Behringwerke AG)] gute therapeutische Erfolge erzielt wurden.

Die vollständige genomische und die cDNA, die für den C1-Inhibitor kodiert, ist bereits kloniert worden (Bock S.C. et al., 1986, Biochemistry 25: 4292; Carter P.E. et al., 1988, Eur J Biochem 173: 163). Verschiedene Varianten des rekombinanten C1-Inhibitors mit Aminosäuremutationen in der P1 und den P3 und/oder P5-Positionen des reaktiven Zentrums als auch Varianten, die aus Patienten mit einem hereditären Angioödem isoliert wurden, sind bereits rekombinant hergestellt worden (Eldering E. et al., 1988, J Biol Chem 263: 11776; Eldering E. et al., 1993, J Biol Chem 267: 7013; Eldering E. et al., 1993, J Clin Invest 91: 1035; US-Patent 5,622,930; Davis A.E. et al., 1992, Nature Genetics 1: 354; Eldering E. et al., 1995, J Biol Chem 270: 2579; Verpy et al., 1995, J Clin Invest 95: 350).

Der C1-Inhibitor gehört zu der großen Familie der Serin-Proteinase Inhibitoren, die auch Serpine genannt werden (Travis J. et al., 1983, Ann Rev Biochem 52: 655; Carrel R.W. et al., 1985, Trends Bioch Sci 10: 20). Auf SDS-Polyacrylamidgelen zeigt der C1-Inhibitor ein Molekulargewicht von etwa 105 kD. Seine Plasmakonzentration liegt bei etwa 270 mg/l (Schapira M. et al., 1985, Complement 2: 111; Nuijens J.H. et al., 1989, J Clin Invest 84: 443). Der C1-Inhibitor ist ein Protein, dessen Plasmaspiegel bei unkomplizierten Infektionen und anderen Entzündungen bis zum zweifachen ansteigen kann (Kalter E.S. et al., 1985 J. Infect, Dis 151: 1019). Die vermehrte Bildung des C1-Inhibitors bei Entzündungen dient wahrscheinlich dem Schutz des Organismus gegen die schädlichen Wirkungen der intravaskulären Aktivierung des Komplementsystems und des Kontaktsystems während der akuten Reaktionen.

Die Serpine reagieren als Inhibitoren durch Bildung bimolekularer Komplexe mit der zu hemmenden Proteinase. Bei diesen Komplexen wird das aktive Zentrum der Proteinase durch das aktive Zentrum des Serpins gebunden und damit inaktiv (Travis J. et al., 1983, Ann Rev Biochem 52: 655). Die Serpine reagieren spezifisch mit bestimmten Proteinasen, wobei diese Spezifität durch die Aminosäuresequenz des reaktiven Zentrums bestimmt wird.

Die vorstehend genannten vielfältigen Wirkungen des C1-Inhibitors konnten jedoch keinen Hinweis auf seine starke Affinität zu HIV geben und insbesondere nicht nahelegen, daß mit Hilfe des C1-Inhibitors eine Abtrennung von HIV aus biologischen Flüssigkeiten wie Blut, Blutplasma oder Blutserum möglich ist. Es war daher ein ganz unerwarteter Befund, daß HIV an C1-Inhibitor bindet und dadurch aus Gemischen, die HIV enthalten, mit Hilfe der nachstehenden Verfahren abgetrennt werden kann.

Gegenstand der Erfindung ist ein Verfahren zum Abtrennen von HIV aus einer Flüssigkeit wie Blut, Blutplasma oder Blutserum, bei dem HIV an einen auf einem Trägermaterial fixierten C1-Esterase-Inhibitor gebunden wird. Dieses Verfahren wird zweckmäßigerweise so durchgeführt, daß der C1-Esterase-Inhibitor an eine in der Affinitätschromatographie einsetzbare, inerte Matrix gebunden wird, über die die von den HIV zu befreiende biologische Flüssigkeit in einer in der Säulenchromatographie üblichen Arbeitsweise gegeben wird.

Als Matrix, auf der der C1-Inhibitor immobilisiert wird, eignen sich Dextrane, Polyacrylamide und Agarose, aber auch andere in der Affinitätschromatographie üblicherweise eingesetzte Träger können für das erfindungsgemäße Verfahren verwendet werden. Dadurch können HIV-freie Blutkonserven gewonnen werden. Es kann aber auch die Viruslast im Blut therapeutisch reduziert werden, wenn vor oder während der Chemotherapie durch eine extrakorporale Blutwäsche HIV an einer mit einem C1-Inhibitor imprägnierten Matrix absorbiert wird.

Eine besonders effektive und schnelle Abtrennung der HIV läßt sich erfindungsgemäß erreichen, wenn man die die HIV enthaltende Flüssigkeit durch eine Fasermasse filtriert, die mit dem C1-Esterase-Inhibitor imprägniert ist. Hierfür hat sich ein Filter bewährt, der aus einem Behälter besteht, in den eine Fasermasse gepackt ist, die mit dem C1-Esterase-Inhibitor imprägniert ist. Die Fasermasse kann entweder aus miteinander verflochtenen oder verwirrten Fasern bestehen oder in Form eines gewebten oder vliesartigen Stoffes vorliegen. Eine besonders wirksame und schnelle Filtration läßt sich dabei mit einem Filter erzielen, der aus mit dem C1-Esterase-Inhibitor imprägnierten Fasern besteht, die einen durchschnittlichen Durchmesser von weniger als 10 mm, vorzugsweise von 0,3 bis 3 mm und eine Schüttdichte von 0,15 bis 0,5 g/cm³ und einen durchschnittlichen Faserabstand von 0,5 bis 0,7 mm aufweisen.

Derartige Filter sind aus der europäischen Patentschrift 0 155 003 bekannt und haben sich zum Abfiltern von Leukozyten aus Blut so hervorragend bewährt, daß sie sich in der Praxis weitgehend durchgesetzt haben. Allerdings sind dort keine imprägnierten Fasern und erst recht nicht mit dem C1-Inhibitor imprägnierte Fasern erwähnt. Setzt man jedoch in einem derartigen Filtersystem mit C1-Inhibitor imprägnierte Fasern ein, dann läßt sich die Affinität dieses Inhibitors zu HIV mit den Vorteilen einer schnellen und effektiven Filtration verbinden, so daß man als Filtrat in einfachster Weise eine von HIV-freie biologische Flüssigkeit gewinnen kann.

Die Vorteile des erfindungsgemäßen Verfahrens werden durch das folgende Beispiel unterstrichen:

### Beispiel

Es wurden folgende Ausgangsmaterialien eingesetzt:
HIV aus Zellkulturen in RPMI-Medium (Titer etwa 10⁴ CCID₅₀)
C1-Inhibitor-Sepharose: 5 mg Ag/ml Sepharose in 2 M NaCl, 20 mM Tris pH 7,2
AT III-Sepharose: 11,1 mg Ag/ml Sepharose in 2 M NaCl, 20 mM Tris pH 7,2

### Versuchsdurchführung:

HIV wurde 1:5 zur Gelsuspension (5 ml zu 20 ml Gelsuspension) zupipettiert, 30 min bei 22° C inkubiert, das Gel niedertourig abzentrifugiert und der Überstand titriert. Als Kontrolle wurde AT III-gekoppelte Sepharose eingesetzt.

Es wurden folgende Ergebnisse erhalten:

| Probe: HIV-Verdünnung | C1-Inhibitor-Sepharose | | AT III-Sepharose | |
|---|---|---|---|---|
| | CCID₅₀* (bestimmt) | CCID₅₀* (theoretisch) | CCID₅₀* (bestimmt) | CCID₅₀* theoretisch) |
| 1:1000 | ≤1,8 | 3,3 | 2,9 | 3,3 |

| | | | | |
|---|---|---|---|---|
| * CCID₅₀log₁₀ (Cell Culture Infective Dose 50%) | | | | |

Der Versuch zeigt, daß HIV an C1-INH bindet; der Kontrollversuch mit AT III-gekoppeltem Gel zeigt, daß die Bindung spezifisch an C1-INH erfolgt und nicht unspezifisch an Gel.

Der Versuch wurde mit etwa 50.000 infektiösen Virionen von HIV im Versuchsansatz durchgeführt; durch 100 mg an Gel gebundenem C1-Inhibitor wurden mindestens etwa 48.000 Virionen aus dem Überstand entfernt.

## Patentansprüche

1. Verfahren zum Abtrennen von HIV aus einer Flüssigkeit, dadurch gekennzeichnet, daß die HIV an einen auf einem Trägermaterial fixierten C1-Esterase-Inhibitor gebunden werden.

2. Verfahren nach Anspruch 1, dadurch gekennzeichnet, daß als Flüssigkeit Blut, Blutplasma oder Blutserum eingesetzt wird.

3. Verfahren nach den Ansprüchen 1 und 2, dadurch gekennzeichnet, daß als Trägermaterial eine in der Affinitätschromatographie einsetzbare, inerte Matrix verwendet wird.

4. Verfahren nach den Ansprüchen 1 bis 3, dadurch gekennzeichnet, daß man die HIV enthaltende Flüssigkeit durch eine Fasermasse filtriert, die mit dem C1-Esterase-Inhibitor imprägniert ist.

5. Filter zum Abtrennen von HIV aus einer Flüssigkeit, dadurch gekennzeichnet, daß er aus einem Behälter besteht, in den eine Fasermasse gepackt ist, die mit dem C1-Esterase-Inhibitor imprägniert ist.

6. Filter nach Anspruch 5, dadurch gekennzeichnet, daß die Fasermasse aus miteinander verflochtenen oder verwirrten Fasern besteht oder in Form eines gewebten oder vliesartigen Stoffes vorliegt.

7. Filter nach den Ansprüchen 5 und 6, dadurch gekennzeichnet, daß die mit dem C1-Esterase-Inhibitor imprägnierten Fasern einen durchschnittlichen Durchmesser von weniger als 10 mm, vorzugsweise von 0,3 bis 3 mm, eine Schüttdichte von 0,15 bis 0,5 g/cm³ und einen durchschnittlichen Faserabstand von 0,5 bis 0,7 mm aufweisen.
